# EUROPÄISCHE PATENTSCHRIFT

(11) **EP 2 825 879 B1**
(45) Veröffentlichungstag und Bekanntmachung des Hinweises auf die Patenterteilung: **22.01.2020**
(21) Anmeldenummer: 13709391.0
(22) Anmeldetag: 06.03.2013
(51) Int. Cl.: G01N 33/00, G01N 27/18, G01N 25/18

(54) **FUNKTIONSÜBERPRÜFUNG VON WÄRMELEITFÄHIGKEITS-GASSENSOREN**
FUNCTIONALITY MONITORING FOR THERMAL CONDUCTIVITY GAS SENSORS
CONTRÔLE DU FONCTIONNEMENT DE CAPTEURS DE GAZ DE CONDUCTIVITÉ THERMIQUE

(30) Priorität: 12.03.2012 EP 12159052
(43) Veröffentlichungstag der Anmeldung: 21.01.2015
(73) Patentinhaber: Mettler-Toledo GmbH, 8606 Greifensee (CH)
(72) Erfinder: OBERLIN, René, CH-5436 Würenlos (CH); MÖBIUS, André, CH-8048 Zürich (CH)
(74) Vertreter: Mettler-Toledo
(86) Internationale Anmeldenummer: PCT/EP2013/054490
(87) Internationale Veröffentlichungsnummer: WO 2013/135539

(56) Entgegenhaltungen:
- WO-A1-2011/035759
- DE-A1- 4 439 715
- DE-B- 1 002 146
- US-A- 5 255 553
- US-B1- 6 623 976

## Beschreibung

Die Erfindung betrifft eine Funktionsüberprüfung von Gassensoren, welche nach dem Prinzip der Wärmeleitfähigkeit arbeiten.

Gassensoren, welche nach dem Prinzip der Wärmeleitfähigkeit arbeiten, können zur Bestimmung von Gasen oder gasförmigen Komponenten in fluiden Medien eingesetzt werden. Als "fluide Medien" werden hier sowohl flüssige als auch gasförmige Medien verstanden. Das zu detektierende Gas oder die zu detektierende gasförmige Komponente, im Folgenden auch als "Target-Gas" bezeichnet, kann im Medium als Gas oder in gelöster Form vorliegen, wobei das fluide Medium neben dem Target-Gas weitere Komponenten oder gelöste Stoffe, wie Feststoffe, Gase oder Flüssigkeiten, enthalten kann. Bestimmt werden zunächst spezifische Wärmeleitfähigkeits-Parameter des Target-Gases, aus welchen weitere Parameter, wie zum Beispiel die Konzentration, der Druck oder der Partialdruck des Target-Gases im fluiden Medium, abgeleitet werden können.

Gassensoren, welche nach dem Prinzip der Wärmeleitfähigkeit arbeiten, sind unter anderem aus EP 0 501 089 A1, EP 0 429 397 A2, EP 0433 741 A1 oder DE 4 439 715 A1 bzw. US 5,255,553 A, DE 10 02 146 B und US 6,623,976 B1 bekannt.

Das Messprinzip basiert auf einer Kombination aus Diffusion eines Target-Gases durch eine Membran mit anschliessender Detektion des Target-Gases durch eine Wärmeleitfähigkeits-Messung. Der eingesetzte Gassensor umfasst eine Membran, welche zur Messung mit dem fluiden Medium in Kontakt gebracht wird. Diese Membran trennt das fluide Medium von einer Messkammer im Gassensor ab, in welcher ein Wärmeleitfähigkeitsfühler angeordnet ist. Die Membran besteht beispielsweise aus einer Polymerschicht oder Polymerfolie, welche für das Target-Gas durchlässig und gleichzeitig flüssigkeitsundurchlässig ist, so dass nur das Target-Gas durch die Membran in die Messkammer gelangen kann. Das eingesetzte Polymer hat eine auf das Target-Gas abgestimmte Permeabilität.

Die Messkammer wird vor jedem Messvorgang mit einem geeigneten Spülgas gespült, um Rückstände einer vorhergehenden Probe des Target-Gases aus der Messkammer zu entfernen. Das Spülgas sollte eine vom Target-Gas abweichende Wärmeleitfähigkeit aufweisen und zudem chemisch inert gegenüber dem Target-Gas und/oder den Materialien des Sensors sein, also mit diesen Substanzen nicht reagieren.

Im Anschluss an diesen Spülvorgang kann das Target-Gas wieder durch die Membran in die Messkammer eindringen. Dabei wird mindestens ein zeitabhängiger Wärmeleitfähigkeits-Parameter mit dem Wärmeleitfähigkeitsfühler gemessen, welcher in Relation zur Konzentration des Target-Gases in der Messkammer und damit im fluiden Medium steht. Aus diesem Parameter kann anschliessend unter Berücksichtigung der Membran-Permeabilität für das Target-Gas zunächst der Partialdruck des Target-Gases bestimmt und daraus dessen Konzentration im fluiden Medium ermittelt werden.

Zudem kann der Gassensor kalibriert werden wie es beispielsweise in EP 0 348 243 A1 offenbart ist.

Eingesetzt werden Gassensoren, welche nach dem Prinzip der Wärmeleitfähigkeit arbeiten, in verschiedenen industriellen Prozessen und Prozessanlagen sowie auch im Labor. Die Einsatzmöglichkeiten reichen dabei von der CO₂-Bestimmung bei der Getränkeherstellung bis zur N₂-, H₂- oder CO₂-Bestimmung in biologischen Prozessen und Bioreaktoren. Weitere Target-Gase welche mit derartigen Gassensoren bestimmt werden können sind beispielsweise Schwefeldioxid (SO₂) oder Schwefelwasserstoff (H₂S).

Insbesondere für den Einsatz in industriellen Prozessanlagen ist es wichtig, dass die Gassensoren zuverlässige und reproduzierbare Ergebnisse gleichbleibender Qualität liefern.

Wie es sich gezeigt hat, werden die Funktionsfähigkeit und damit die Zuverlässigkeit der Gassensoren durch verschiedene Fehlzustände beeinflusst, wie Fehler in der Spülgaszufuhr und/oder das Eindringen von Feuchtigkeit in das Innere des Sensors.

Diese Fehlzustände führen zu fehlerhaften Messungen und können sogar zu einem Ausfall des Gassensors führen.

Daraus ergibt sich als Aufgabe die Bereitstellung eines Verfahrens zur Funktionsüberprüfung eines Gassensors, welcher nach dem Prinzip der Wärmeleitfähigkeit arbeitet, um Fehlzustände des Gassensors bereits im Betrieb und insbesondere on-line zu erkennen.

Gelöst wird diese Aufgabe durch ein Verfahren zur on-line Funktionsüberprüfung eines Gassensors, welcher nach dem Prinzip der Wärmeleitfähigkeit arbeitet. Der Gassensor umfasst eine Messkammer mit einem Spülgasanschluss, einen in der Messkammer angeordneten Wärmeleitfähigkeitsfühler zur Bestimmung eines oder mehrerer Messwerte, einen Temperaturfühler und eine Membran, welche im Betrieb die Messkammer von einem fluiden Medium abtrennt und für ein Target-Gas durchlässig ist. Das Verfahren zur Funktionsüberprüfung des Gassensors umfasst mehrere Schritte.

Zunächst wird ein Kalibrier-Zyklus in einem Kalibriermedium durchgeführt und eine Kalibrier-Baseline ermittelt. Das Kalibriermedium ist ein fluides Medium mit einer bekannten Konzentration des Target-Gases. Ein Kalibrier-Zyklus besteht aus mindestens einem Spülvorgang und einem Messvorgang. Während eines Messvorgangs und/oder eines Spülvorgangs kann mindestens ein Messwert mit dem Wärmeleitfähigkeitsfühler erfasst und daraus eine entsprechende Kalibrier-Baseline ermittelt werden.

Anschliessend wird ein Mess-Zyklus in einem Messmedium durchgeführt und aus den erhaltenen Messwerten eine entsprechende Mess-Baseline ermittelt. Das Messmedium ist ein fluides Medium mit unbekannter Konzentration eines Target-Gases. Ein Mess-Zyklus umfasst mindestens einen Spülvorgang und einen Messvorgang, welche nacheinander durchgeführt werden. Zur Aufnahme von Messwerten oder einer Messwertreihe wird vor oder im Anschluss an den Messvorgang ein Spülvorgang durchgeführt, so dass die Messkammer zu Beginn eines Messvorgangs im Wesentlichen frei von Rückständen ist.

In einem Spülvorgang wird die Messkammer mit einem Spülgas gespült und so mögliche Rückstände aus der Messkammer entfernt. Anschliessend kann ein Messvorgang durchgeführt werden, bei dem die Membran des Gassensors mit einem fluiden Medium in Kontakt gebracht wird, so dass im fluiden Medium enthaltenes Target-Gas durch die Membran in die Messkammer diffundieren kann. In der Messkammer vorhandenes Spülgas mischt sich dabei mit dem Target-Gas oder wird durch dieses verdrängt. Mit dem in der Messkammer angeordneten Wärmeleitfähigkeitsfühler können ein oder mehrere Messwerte bestimmt werden. Die Messwerte können zu vorgegebenen Zeitpunkten, mit vorgegebenen Zeitabständen oder kontinuierlich erfasst werden.

Aus mehreren während eines Kalibrier- oder Mess-Zyklus als Funktion der Zeit bestimmten Messwerten kann eine Kalibrier- oder Mess-Baseline ermittelt werden. Unter Einbeziehung der Kalibrier- und der Mess-Baseline kann ein Baseline-Vergleichswert ermittelt werden, welcher dann mit einem vorgegebenen Baseline-Grenzwert verglichen wird. Wenn der Betrag des Baseline-Vergleichswerts grösser ist als der Baseline-Grenzwert wird eine erste Fehlermeldung erzeugt. Die erste Fehlermeldung zeigt eine Fehlfunktion in der Spülgaszufuhr an.

Wie es sich überraschenderweise gezeigt hat, kann bereits durch einen Baseline-Vergleich zwischen Kalibrier-Zyklus und Mess-Zyklus eine Aussage über eine Fehlfunktion der Spülgaszufuhr getroffen werden, so dass die Spülgaszufuhr manuell oder automatisch überprüft werden kann. Als Baseline wird hier die Messspannung als Funktion der während des Spülvorgangs ermittelten Spannungswerte bezeichnet. Für die Kalibrier-Baseline werden die während des Kalibriervorgangs ermittelten Baseline-Werte herangezogen, welche eine Funktion der während des Spülvorgangs innerhalb des Kalibriervorgangs ermittelten Spannungswerte sind. Für die Mess-Baseline werden die während des Messvorgangs ermittelten Baseline-Werte verwendet, welche eine Funktion der während des Spülvorgangs innerhalb des Messvorgangs ermittelten Spannungswerte sind. Dem Benutzer kann die Fehlfunktion zudem angezeigt werden, beispielsweise als elektronisches, akustisches und/oder optisches Signal. Auf diese Weise können Messwerte mit einer hohen Reproduzierbarkeit und einer hohen Qualität erzeugt werden.

Das Verfahren kann zudem Schritte umfassen, mit welchen überprüft werden kann, ob Feuchtigkeit in die Messkammer eingedrungen ist, und welche zur Erzeugung von einer oder mehreren weiteren Fehlermeldungen führt. Durch das Eindringen von Feuchtigkeit in die Messkammer können die Messwerte verfälscht werden.

Dazu wird ein Mess-Zyklus in einem Messmedium durchgeführt, welcher einen Spül- und einen Messvorgang umfasst. Während des Messvorgangs wird eine Messspannung mit einer elektronischen Messschaltung des Gassensors erfasst, welche eine Mass für die zu messende Wärmeleitfähigkeit darstellt. Diese Messspannung wird mit einem vorgegebenen Spannungsbereich verglichen. Wenn die Messspannung ausserhalb des vorgegebenen Spannungsbereichs liegt, wird eine weitere Fehlermeldung erzeugt. Liegt die Messspannung innerhalb des vorgegebenen Spannungsbereichs, so kann zusätzlich eine Messstromstärke aus dem Spannungsabfall über dem Messwiderstand bzw. über dem entsprechenden Shunt-Widerstand der Messschaltung bestimmt werden. Durch Vergleich der Messstromstärke mit einer vorgegebenen Messstromstärke wird ein erster Kontrollwert ermittelt. Zudem wird eine Kontrollstromstärke über eine Kontrollschaltung ermittelt. Die Messschaltung umfasst die Kontrollschaltung. Die Kontrollstromstärke wird aus dem Spannungsabfall über dem entsprechenden Shunt-Widerstand der Kontrollschaltung bestimmt. Ein zweiter Kontrollwert kann durch Vergleich der Messstromstärke mit der Kontrollstromstärke ermittelt werden.

Der erste und/oder der zweite Kontrollwert können anschliessend mit einem ersten Kontroll-Grenzwert verglichen werden. Wenn der erste und/oder der zweite Kontrollwert grösser als der Kontroll-Grenzwert sind, dann wird eine zweite Fehlermeldung erzeugt, welche eine Fehlfunktion des Gassensors aufgrund von in die Messkammer eingedrungener Feuchtigkeit anzeigt.

Dieses Verfahren kann weiterhin einen Vergleich des zweiten Kontrollwerts mit einem dritten Kontroll-Grenzwert umfassen, wenn der zweite Kontrollwert grösser ist als der zweite Kontroll-Grenzwert. Wenn der zweite Kontrollwert grösser als der dritte Kontroll-Grenzwert ist, so kann eine dritte Fehlermeldung erzeugt werden, welche einen Ausfall des Gassensors aufgrund in die Messkammer eingedrungener Feuchtigkeit anzeigt.

Die in die Messkammer eingedrungene Feuchtigkeit kann alternativ oder zusätzlich auch über die folgenden Verfahrensschritte bestimmt und dem Anwender zur Anzeige gebracht werden. Dazu wird ein Mess-Zyklus in einem Messmedium durchgeführt und eine Messspannung während des Messvorgangs mit einer elektronischen Messschaltung des Gassensors bestimmt. Anschliessend wird eine Messstromstärke aus dem Spannungsabfall über dem Messwiderstand bzw. über dem Shuntwiderstand der Messschaltung bestimmt. Durch Nachregeln eines regelbaren Widerstands oder der Versorgungsspannung der elektronischen Messschaltung kann die Messstromstärke an eine vorgegebene Messstromstärke angepasst werden. Die dafür notwendige Änderung des regelbaren Widerstands oder der Versorgungsspannung wird als Stellwert ermittelt. Dieser Stellwert wird anschliessend mit einem vorgegebenen ersten Kontroll-Stellwert verglichen. Wenn der Stellwert grösser ist als der Kontroll-Stellwert kann eine zweite Fehlermeldung erzeugt werden, welche eine Fehlfunktion des Gassensors anzeigt.

Weiterhin kann zudem ein Vergleich des Stellwerts mit einem zweiten Kontroll-Stellwert durchgeführt werden, wenn der Stellwert grösser ist als der erste Kontroll-Stellwert. Wenn der Stellwert grösser ist als der erste und grösser als der zweite Kontroll-Stellwert ist, so kann eine dritte Fehlermeldung erzeugt werden, welche einen Ausfall des Gassensors aufgrund in die Messkammer eingedrungener Feuchtigkeit anzeigt.

Ein weiterer Aspekt des erfindungsgemässen Verfahrens betrifft die Überprüfung der Membran des Gassensors. Die Membran schliesst die Messkammer des Gassensors gegen das fluide Medium ab und ist zudem für das Target-Gas durchlässig. Die Membran ist somit semipermeabel und hat eine definierte Permeabilität. Wenn sich die Durchlässigkeit der Membran ändert, beispielsweise wenn die Membran mechanische Fehlstellen, wie Risse oder Löcher aufweist, oder wenn die Membran defekt ist, beispielsweise aufgrund von veränderten Porengrössen, dann hat dieses einen direkten Einfluss auf die Messergebnisse. Die Messergebnisse können durch eine defekte Membran verfälscht werden, da beispielsweise neben dem Target-Gas Störkomponenten in die Messkammer gelangen können oder die Diffusion des Target-Gases in die Messkammer behindert oder aber stark erhöht werden kann. Daher ist es wünschenswert, wenn auch die Funktionsfähigkeit der Membran eines Gassensors, insbesondere on-line, überprüft werden kann.

Dazu kann ein Mess-Zyklus mit einem Gassensor, welcher nach dem Prinzip der Wärmeleitfähigkeit arbeitet, durchgeführt werden und die Messspannung als Funktion der Zeit während des Messvorgangs bestimmt werden. Das zeitliche Verhalten der Messspannung während des Messvorgangs kann anschliessend mit einem vorgegebenen ersten Membran-Grenzwertbereich verglichen werden. Während eines folgenden Spül-Zyklus kann eine Spül-Messspannung während des Messvorgangs des Spül-Zyklus bestimmt werden und das zeitlichen Verhalten der Spül-Messspannung mit einem vorgegebenen zweiten Membran-Grenzwertbereich verglichen werden.

Wenn beide Spannungen, die Messspannung und die Spül-Messspannung, ausserhalb ihrer jeweiligen Grenzwertbereiche, dem ersten Membran-Grenzwertbereich bzw. dem zweiten Membran-Grenzwertbereichs liegen, so kann eine vierte Fehlermeldung erzeugt werden, welche eine Fehlfunktion der Membran im Gassensor anzeigt.

Auf diese Weise kann der Benutzer bereits im laufenden Betrieb des Gassensors über Probleme mit der Membran und/oder eine fehlerhafte Membran informiert werden und Massnahmen zum Austausch der Membran oder des Gassensors ergreifen.

Die erste, zweite, dritte und/oder vierte Fehlermeldung kann in einer Steuereinheit des Gassensors registriert werden. Weiterhin kann die erste, zweite, dritte und/oder vierte Fehlermeldung dem Benutzer elektronisch, optisch und/oder akustisch angezeigt werden.

Auf diese Weise kann eine in der Steuereinheit des Gassensors registrierte Fehlermeldung, z.B. eine Kennzeichnung der mit einer fehlerhaften Membran bestimmten Messwerte bewirken, so dass diese bei einer späteren Auswertung nicht berücksichtigt werden, oder sogar eine Abschaltung des Gassensors hervorrufen. Der Benutzer kann zudem bereits im laufenden Betrieb des Gassensors über eine Fehlfunktion des Gassensors informiert werden und Massnahmen zur Behebung der Fehlfunktion treffen oder einleiten, beispielsweise den Austausch von Komponenten, wie der Membran, die Überprüfung und Veränderung der Einstellungen der Spülgaszufuhr, den Austausch von Dichtungen und/oder den Austausch des gesamten Gassensors.

Ein Gassensor zur Durchführung des erfindungsgemässen Verfahrens zur on-line Funktionsüberprüfung umfasst eine Messkammer mit einem Spülgasanschluss, einen in der Messkammer angeordneten Wärmeleitfähigkeitsfühler zur Bestimmung eines oder mehrerer Messwerte, und eine Membran, welche im Betrieb die Messkammer von einem fluiden Medium abtrennt und für ein Target-Gas durchlässig ist, welches im fluiden Medium vorliegt, wobei beim Durchführen eines Spül-Zyklus und/oder eines Mess-Zyklus mindestens eine erste, zweite und/oder dritte Fehlermeldung vom Gassensor erzeugbar ist, welche mindestens eine Fehlfunktion des Gassensors aufgrund einer fehlerhaften Spülgaszufuhr, eingedrungener Feuchtigkeit und/oder der Membran anzeigt.

Weiterhin kann der Gassensor eine Steuereinheit umfassen, welche ganz oder teilweise in einem Sensorkörper des Gassensors angeordnet ist, wobei die Steuereinheit die erste, zweite, dritte und/oder vierte Fehlermeldung registriert und/oder verarbeitet.

Die Steuereinheit umfasst zudem einen Kontrollschaltkreis, welcher Fehlströme detektiert kann, die durch in den Sensorkörper eingedrungene Feuchtigkeit entstehen.

Verschiedene Ausführungsbeispiele eines erfindungsgemässe Verfahren sowie eines zur Durchführung des Verfahrens geeigneten Gassensors werden anhand der folgenden Figuren näher beschrieben, wobei gleiche Elemente mit gleichen oder ähnlichen Bezugszeichen versehen sind. Die Figuren zeigen:
- Fig. 1: Eine Explosionsdarstellung eines Gassensors, welcher nach dem Prinzip der Wärmeleitfähigkeit arbeitet;
- Fig. 2: eine stark schematisierte Schnittzeichnung durch den Messstutzen eines Gassensors;
- Fig. 3: ein stark vereinfachtes Schaltschema eines Gassensors mit einer Kontrollschaltung zur Überprüfung von Fehlströmen;
- Fig. 4: ein Spannungs-Zeit-Diagramm für einen typischen Kalibrier- oder Mess-Zyklus zur Bestimmung einer Basislinie;
- Fig. 5: ein Diagramm, welches den gemessenen CO2-Partialdruck und die ermittelte Baseline in Abhängigkeit des Spülgasflusses und der Zeit zeigt.

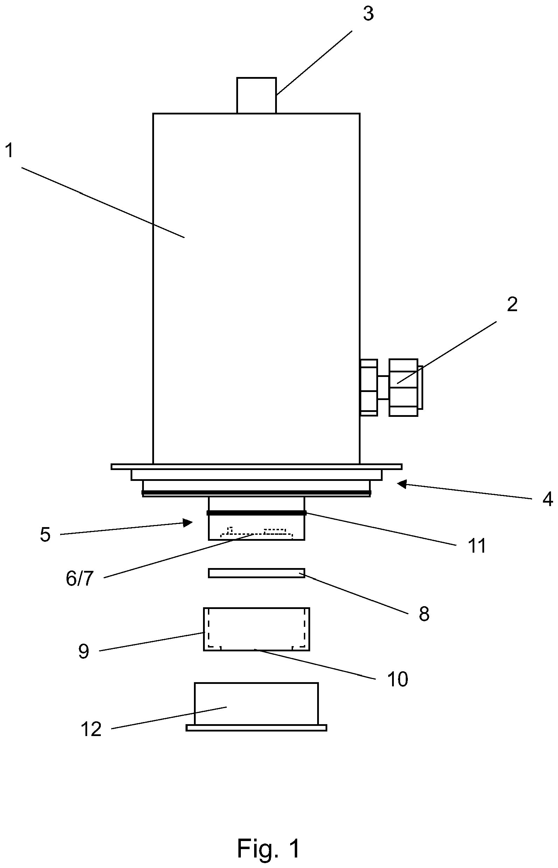

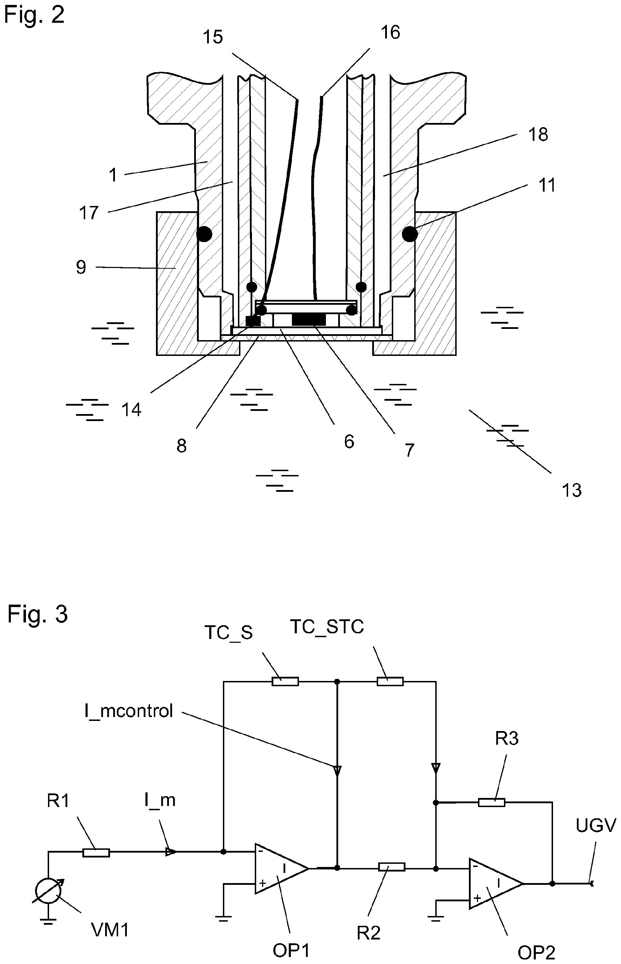

Figur 1 zeigt eine schematisierte Explosionsdarstellung eines nach dem Prinzip der Wärmeleitfähigkeit arbeitenden Gassensors. Der Gassensor umfasst einen Sensorkörper 1, welcher einen Spülgasanschluss 2 und einen Anschluss 3 zur Verbindung des Gassensors mit einem Transmitter und/oder einer Prozessleitstelle umfasst. Im Sensorkörper 1 sind unter anderem die Messelektronik und die Steuerelektronik sowie die zugehörigen Schaltkreise angeordnet, welche hier nicht zu sehen sind. An seinem mediumseitigen Ende, welches im Betrieb mit einem fluiden Medium in Kontakt steht, weist der Gassensor einen Flansch 4 zum Verbinden des Gassensors mit einem Behältnis auf. Weiterhin ist am mediumseitigen Ende des Gassensors ein im Betrieb in das fluide Medium hineinragender Messstutzen 5 zu erkennen, in welchem eine Messkammer 6 mit einem Wärmeleitfähigkeitsfühler 7 angeordnet ist, s. a. Figur 2.

Die Messkammer 6 wird von einer Membran 8 bedeckt, welche mit einer Kappe 9 am Messstutzen 7 befestigt wird. Die Kappe 9 weist eine Öffnung 10 auf, durch die das fluide Medium 13 mit der Membran 8 in Kontakt treten kann. Die Zusammensetzung und der Aufbau der Membran 8 sind auf das interessierende Target-Gas abgestimmt. Die Membran 8 kann beispielsweise ein keramisches Material, einen Komposit-Werkstoff und/oder einen polymeren Werkstoff umfassen. Eine Membran 8, welche insbesondere für die CO₂ Messung geeignet ist, kann beispielsweise einen Komposit-Werkstoff umfassen, welcher aus einem Polymerfilm, wie PTFE, besteht, der zur mechanischen Verstärkung mit einer geeigneten rigiden Gitterstruktur, wie einem Metallnetz, fest verbunden ist. Zwischen der Kappe 9 und dem Messstutzen 7 ist zudem eine Dichtung 11, hier in Form eines O-Rings, angeordnet, um die Messkammer 6 gegenüber dem fluiden Medium abzudichten.

Für die Lagerung oder den Transport kann der Messstutzen 5 und insbesondere die Membran 8 zudem mit einer Abdeckung 12 geschützt werden.

Figur 2 zeigt stark schematisiert einen Schnitt durch den Messstutzen 5 im zusammengebauten Zustand des Gassensors ohne Abdeckung. Wie hier zu erkennen ist, taucht der Messstutzen 5 im Betrieb in ein fluides Medium 13 ein, welches in einem geeigneten Behältnis angeordnet ist. In dem fluiden Medium 13 befindet sich ein Target-Gas, welches in dem fluiden Medium 13 gelöst oder mit diesem vermischt ist. Das Target-Gas kann durch die Membran 8 in die Messkammer 6 eindringen, da die Membran 8 selektiv permeabel für das Target-Gas ist.

Am Boden der Messkammer 6 sind ein Wärmeleitfähigkeitsfühler 7 und ein Temperaturfühler 14 angeordnet, welche jeweils über hier nur angedeutete Leitungen 15, 16 mit der im Gassensor angeordneten Steuereinheit verbunden sind. Über die mit dem Temperaturfühler 14 bestimmte Temperatur kann eine Temperaturkompensation des mit dem Wärmeleitfähigkeitsfühler 7 bestimmten Messwerts durchgeführt werden.

Weiterhin umfasst die Messkammer 6 eine Gas-Zuleitung 17 und einen Gas-Auslass 18. Über die Gas-Zuleitung 17 kann die Messkammer 6 mit einem Spülgas geflutet werden, um Rückstände aus der Messkammer 6 zu entfernen. Über den Gas-Auslass 18 können das Spülgas und/oder das auszutreibende Target-Gas aus der Messkammer 6 entweichen. Der Gas-Auslass 18 stellt zudem sicher, dass ein Druckaufbau in der Messkammer 6 verhindert wird.

Figur 3 zeigt ein stark vereinfachtes Schaltschema einer elektronischen Messschaltung des Gassensors mit einer Kontrollschaltung.

Der Gassensor wird mit einem konstanten Versorgungsstrom I_m versorgt, welcher für die jeweilige Messwertbestimmung fest vorgegeben ist. Der Strom I_m wird durch eine voreingestellte Versorgungsspannung VM1 und einen variablen Widerstand R1 eingestellt.

Weiterhin kann der Strom I_m auch als Teil der Kontrollschaltung regelbar sein, um die Funktion des Gassensors on-line zu überprüfen. In diesem Fall umfasst die Messschaltung entweder einen regelbaren Widerstand R1, oder eine regelbare Versorgungsspannung VM1.

Dieser konstante Versorgungsstrom I_m ermöglicht die für die Messung wichtige Bestimmung des Widerstands TC_S des Wärmeleitfähigkeitsfühlers über die Ausgangsspannung eines ersten Operationsverstärkers OP1. Die Ausgangsspannung des ersten Operationsverstärkers OP1 wird noch über einen weiteren Schaltungsteil mit einem zweiten Widerstand R2 und dem Temperaturfühler, hier als Widerstand TC_STC bezeichnet, sowie einen dritten Widerstand R3 und einen zweiten Operationsverstärkers OP2 einerseits temperaturkompensiert und andererseits verstärkt. Die Ausgangsspannung des zweiten Operationsverstärkers OP2 ist die eigentliche Messspannung UGV welche proportional zur zu messenden Wärmeleitfähigkeit ist.

Mit dem Gassensor können Kalibrier- und Mess-Zyklen durchgeführt werden, wobei jeder Zyklus jeweils aus mindestens einem Spülvorgang und einem Messvorgang besteht. Ein Kalibrier-Zyklus wird in einem Kalibriermedium mit bekannter Target-Gas-Konzentration und ein Messvorgang in einem Messmedium mit unbekannter Target-Gas-Konzentration als fluides Medium durchgeführt.

Zunächst wird die Messkammer mit einem geeigneten Spülgas gespült, dieser Spülvorgang kann ein oder mehrfach durchgeführt werden. Anschliessend wird der Gassensor, insbesondere der Messstutzen, mit dem entsprechenden fluiden Medium in Kontakt gebracht. Das im fluiden Medium enthaltene Target-Gas diffundiert durch die Membran in die Messkammer, wo mittels des Wärmeleitfähigkeitsfühlers die Wärmeleitfähigkeit in der Messkammer als Messwert ermittelt wird. Die Messwerte können während eines Messvorgangs als Einzelwerte, in vorgegebenen zeitlichen Abständen oder kontinuierlich erfasst werden. Aufgrund von mit dem Temperaturfühler bestimmten Temperaturwerten können temperaturkompensierte Messwerte ermittelt werden. Als Spülgas können verschiedene Gase eingesetzt werden, welche unter anderem Pressluft, Stickstoff (N₂), Kohlendioxid (CO₂) oder ein Edelgas, wie Argon oder Helium, umfassen. Die Auswahl des Spülgases wird unter anderem durch die Zusammensetzung des Messmediums, beispielsweise das Vorliegen von Störgasen, und/oder durch das zu bestimmende Target-Gas beeinflusst.

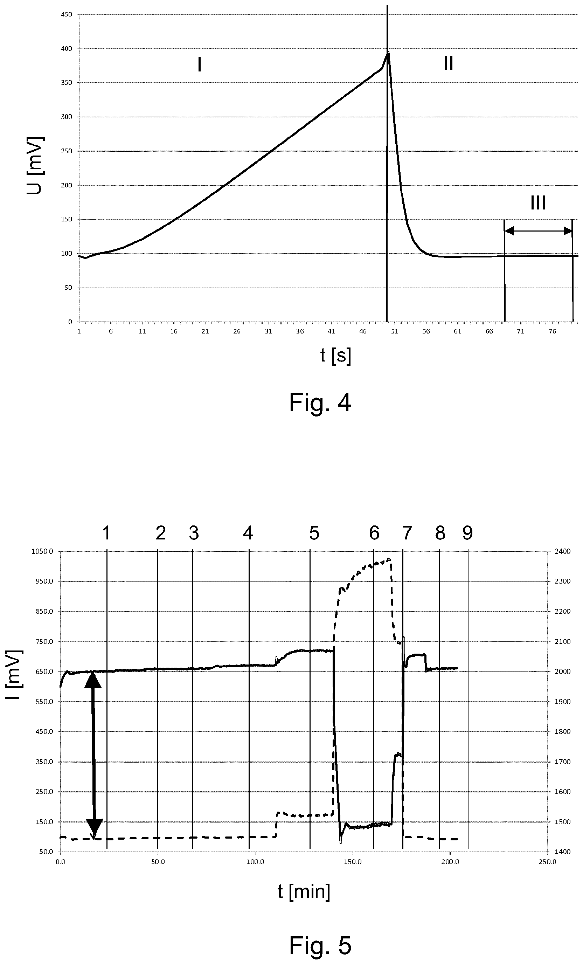

Beispiele zur Umsetzung des erfindungsgemässen Verfahrens sind in den Figuren 4 und 5 gezeigt.

Figur 4 zeigt eine typische Spannungs-Zeit-Kurve der Messspannung eines Mess- oder Kalibrier-Zyklus. Die Kurve wurde mit einem erfindungsgemässen CO₂-Gassensor in reinem CO₂-Gas als Messmedium und Luft als Spülgas aufgenommen. Während des Messvorgangs I diffundiert das Target-Gas aus dem fluiden Medium in die Messkammer, so dass sich mit der Zeit die Konzentration des Target-Gases in der Messkammer erhöht und damit auch die erfasste Wärmeleitfähigkeit, welche proportional zur gezeigten Messspannung UGV ist. Nach Abschluss des Messvorgangs, welcher hier mehrere Messwerte umfasst, wird ein Spülvorgang II eingeleitet, bei welchem das Spülgas durch die Messkammer geleitet und so das Target-Gas aus der Messkammer ausgetrieben wird. Die Messkammer kann beispielsweise solange gespült werden, bis die Messwerte des Wärmeleitfähigkeitsfühlers im Wesentlichen stabil sind. Durch Mittelwertbildung über die erfasste Messspannung UVG im Bereich III der Kurve kann anschliessend die Baseline bestimmt werden. Bereich III umfasst hier die letzten vier Sekunden des Spülvorgangs eines Kalibrier- oder Mess-Zyklus in welchen der Gassensor im Wesentlichen stabile Messwerte liefert. Wenn der Gassensor in Kontakt mit einem Kalibriermedium ist, kann auf diese Weise eine Kalibrier-Baseline oder wenn der Gassensor mit einem Messmedium in Kontakt ist kann eine Mess-Baseline bestimmt werden. Die Baseline umfasst die Kalibrier- oder Messspannung als Funktion der während des Spülvorgangs ermittelten Spannungswerte.

In Figur 5 ist das zeitliche Verhalten der Baseline-Charakteristik in Abhängigkeit des Spülgasflusses gezeigt. Die Messungen wurden mit einem erfindungsgemässen CO₂-Gassensors mit Luft als Spülgas aufgenommen. Als Messmedium wurde reines CO₂-Gas mit einem CO₂-Partialdruck von etwa 2000 mbar verwendet. Während den Messungen wird der Spülgasfluss von etwa 70 ml/Min auf etwa 0.2 - 0.5 ml/Min reduziert und anschliessend wieder auf den Anfangswert erhöht. Die verschiedenen Spülgasflüsse sind mit vertikalen Linien im Diagramm gekennzeichnet, wobei etwa folgende Spülgasflüsse eingestellt wurden: 1: 70 ml/Min, 2: 50 ml/Min, 3: 30 ml/Min, 4: 10-20 ml/Min, 5: 1-5 ml/Min, 6: 0.2-0.5 ml/Min, 7: 0.3-1.2 ml/Min, 8: 3-14 ml/Min, 9: 70 ml/Min.

Die Mess-Baseline-Kurve, hier als unterbrochene Linie dargestellt, zeigt zunächst den Wert einer während eines Kalibrier-Zyklus ermittelte Kalibrier-Baseline von etwa 93 mV, bei einem optimalen Spülgasfluss von etwa 70 ml/Min. Der Doppelpfeil deutet den Zeitpunkt an, zu dem der Sensor kalibriert und die Kalbrier-Baseline erfasst wurde. Im Laufe der weiteren Messungen wurde der Spülgasfluss gestört, indem dieser reduziert wurde, um eine fehlerhafte Spülgaszufuhr zu simulieren. Bereits Abweichungen im Spülgasfluss von etwa 1 bis etwa 5 ml/Min führen zu signifikanten Abweichungen des CO₂-Messwerts, welcher als CO₂-Partialdruck als durchgezogene Kurve dargestellt ist. Die ungünstige Veränderung des Spülgasflusses lässt sich auch an der Kurve der Mess-Baseline ablesen, da sich diese bereits bei einem auf ca. 10-20 ml/Min erniedrigtem Spülgasfluss (Linie 4) um etwa 80 mV auf etwa 173 mV erhöht. Eine weitere Reduktion des Spülgasflusses auf etwa 0.2 bis etwa 0.5 ml/Min (Linie 6), was im Wesentlichen einem Spülgasunterbruch entspricht, führt zu einem Anstieg der Mess-Baseline auf etwa 990 mV und gleichzeitig einem dramatischen Einbruch des CO₂-Messwerts. Sobald der Spülgasfluss wieder auf den anfänglichen optimalen Wert von etwa 70 ml/Min (Linie 9) eingestellt wurde, zeigt sich im Diagramm, dass die Werte der Mess-Basislinie wie auch der gemessene CO₂-Partialdruck wieder den Anfangswerten entsprechen.

Eine Fehlfunktion des Gassensors aufgrund einer fehler- oder mangelhaften Spülgaszufuhr kann somit anhand des zuvor beschriebenen Verfahrens ermittelt werden, in dem ein Baseline-Grenzwert für die Abweichung der Mess-Baseline von der Kalibrier-Baseline von etwa 50 mV festgelegt wird. Der Wert des Baseline-Grenzwerts kann wie hier gezeigt einfach für jeden Sensortyp und/oder jedes Target-Gas ermittelt werden. Nach Erkennen der fehlerhaften Spülgaszufuhr, kann die Messung nach Überprüfung und Anpassung der Spülgaszufuhr fortgesetzt werden.

Obwohl die Erfindung durch die Darstellung spezifischer Ausführungsbeispiele beschrieben worden ist, ist es offensichtlich, dass zahlreiche weitere Ausführungsvarianten in Kenntnis der vorliegenden Erfindung geschaffen werden können, beispielsweise indem die Merkmale der einzelnen Ausführungsbeispiele miteinander kombiniert und/oder einzelne Funktionseinheiten der Ausführungsbeispiele ausgetauscht werden.

### Bezugszeichenliste

- 1: Sensorkörper
- 2: Spülgasanschluss
- 3: Anschluss
- 4: Flansch
- 5: Messstutzen
- 6: Messkammer
- 7: Wärmeleitfähigkeitsfühler
- 8: Membran
- 9: Kappe
- 10: Öffnung
- 11: Dichtung
- 12: Abdeckung
- 13: Fluides Medium
- 14: Temperaturfühler
- 15: Leitung
- 16: Leitung
- 17: Zuleitung
- 18: Gasauslass
- I_m: Versorgungsstrom
- VM1: Versorgungsspannung
- R1, R2, R3: Widerstand
- OP1, OP2: Operationsverstärker
- UVG: Messspannung
- TC_S: Widerstand des Wärmeleitfähigkeitsfühlers
- TC_STC: Widerstand des Temperaturfühlers
- I: Messvorgang
- II: Spülvorgang
- III: Baseline

## Patentansprüche

1. Verfahren zur Funktionsüberprüfung eines Gassensors, welcher nach dem Prinzip der Wärmeleitfähigkeit arbeitet, wobei der Gassensor eine Messkammer (6) mit einem Spülgasanschluss (2), einen in der Messkammer (6) angeordneten Wärmeleitfähigkeitsfühler (7) zur Bestimmung eines oder mehrerer Messwerte, einen Temperaturfühler (14), und eine Membran (8), welche im Betrieb die Messkammer (6) von einem fluiden Medium (13) abtrennt und für ein Target-Gas durchlässig ist, umfasst; und wobei das Verfahren die folgende Schritte umfasst:
a. Durchführen eines Kalibrier-Zyklus in einem Kalibriermedium, das ein fluides Medium mit bekannter Target-Gas-Konzentration ist, wobei der Kalibrier-Zyklus einen Spülvorgang und einen anschliessenden Messvorgang umfasst, und Ermitteln einer Kalibrier-Baseline, welche die Kalibrier-Spannung als Funktion der während des Spülvorgangs ermittelten Spannungswerte umfasst;
b. Durchführen eines Mess-Zyklus in einem Messmedium, das ein fluides Medium mit unbekannter Target-Gas-Konzentration ist, wobei der Mess-Zyklus mindestens einen Spülvorgang und einen anschliessenden Messvorgang umfasst, und Ermitteln einer Mess-Baseline, welche die Mess-Spannung als Funktion der während des Spülvorgangs ermittelten Spannungswerte umfasst;
c. Berechnen eines Baseline-Vergleichswerts unter Einbeziehung der Kalibrier- oder der Mess-Baseline;
d. Vergleichen des Baseline-Vergleichswerts mit einem vorgegebenen Baseline-Grenzwert, und wenn der Betrag des Baseline-Vergleichswerts grösser ist als der Baseline-Grenzwert, Erzeugen einer ersten Fehlermeldung, welche eine Fehlfunktion in der Spülgaszufuhr anzeigt,
wobei ein Spülvorgang umfasst, dass die Messkammer (6) mit einem Spülgas gespült wird; wobei ein Messvorgang umfasst, dass die Membran (8) des Gassensors mit dem fluiden Medium in Kontakt gebracht wird, dass das im fluiden Medium enthaltene Target-Gase durch die Membran (8) in die Messkammer (6) diffundiert, und dass mit einem in der Messkammer (6) angeordneten Wärmeleitfähigkeitsfühler (7) ein oder mehrere Messwerte bestimmt werden.

2. Verfahren nach Anspruch 1, **dadurch gekennzeichnet, dass** dieses ferner folgende Schritte umfasst:
• Durchführen eines Mess-Zyklus in einem Mess-Medium und während des Messvorgangs Bestimmen einer Messspannung mit einer elektronischen Messschaltung des Gassensors;
• Vergleichen der Messspannung mit einem vorgegebenen Spannungsbereich und
i. Erzeugen einer weiteren Fehlermeldung, wenn die Messspannung ausserhalb des Spannungsbereichs liegt oder
ii. Ermitteln einer Messstromstärke, wenn die Messspannung innerhalb des Spannungsbereichs liegt, und Ermitteln eines ersten Kontrollwerts durch Vergleich der Messstromstärke mit einer vorgegebenen Messstromstärke;
Bestimmen einer Kontrollstromstärke über eine Kontrollschaltung, welche ein Teil der Messschaltung ist, und Ermitteln eines zweiten Kontrollwerts durch Vergleich der Messstromstärke mit der Kontrollstromstärke ;
Vergleichen des ersten Kontrollwerts mit einem ersten Kontroll-Grenzwert und des zweiten Kontrollwerts mit einem zweiten Kontroll-Grenzwert, und
Erzeugen einer zweiten Fehlermeldung, welche eine Fehlfunktion des Gassensors anzeigt, wenn der erste Kontrollwert grösser als der erste Kontroll-Grenzwert und/oder der zweite Kontrollwert grösser als der zweite Kontroll-Grenzwert sind.

3. Verfahren nach Anspruch 2, **dadurch gekennzeichnet, dass** dieses ferner folgende Schritte umfasst:
• Vergleichen des zweiten Kontrollwerts mit einem dritten Kontroll-Grenzwert, wenn der zweite Kontrollwert grösser ist als der zweite Grenzwert; und
Erzeugen einer dritten Fehlermeldung, welche eine Fehlfunktion Ausfall des Gassensors anzeigt, wenn der zweite Kontrollwert grösser als der dritte Kontrollgrenzwert ist.

4. Verfahren nach Anspruch 1, **dadurch gekennzeichnet, dass** dieses ferner folgende Schritte umfasst:
• Durchführen eines Mess-Zyklus in einem Mess-Medium und Bestimmen einer Messspannung während des Messvorgangs mit einer elektronischen Messschaltung des Gassensors;
• Ermitteln einer Messstromstärke, und Nachregeln eines regelbaren ersten Widerstands oder der Versorgungsspannung der elektronischen Messschaltung unter Ermittlung eines Stellwerts, so dass die Messstromstärke einer vorgegebenen Messstromstärke entspricht;
• Vergleichen des Stellwerts mit einem vorgegebenen ersten Kontroll-Stellwert und Erzeugen einer zweiten Fehlermeldung, welche eine Fehlfunktion des Gassensors anzeigt, wenn der Stellwert grösser ist als der erste Kontroll-Stellwert.

5. Verfahren nach Anspruch 4, **dadurch gekennzeichnet, dass** dieses ferner folgende Schritte umfasst:
• Vergleichen des Stellwerts mit einem zweiten Kontroll-Stellwert, wenn der Stellwert grösser ist als der erste Kontroll-Stellwert; und
• Erzeugen einer dritten Fehlermeldung, welche einen Ausfall des Gassensors anzeigt, wenn der Stellwert grösser als der erste und grösser als der zweite Kontroll-Stellwert ist.

6. Verfahren nach einem der Ansprüche 1 bis 5, **dadurch gekennzeichnet, dass** dieses ferner folgende Schritte umfasst:
• Durchführen eines Mess-Zyklus und Bestimmen der Messspannung als Funktion der Zeit während des Messvorgangs;
• Vergleichen des zeitlichen Verhaltens der Messspannung mit einem vorgegebenen ersten Membran-Grenzwertbereich;
• Durchführen eine Spül-Vorgangs und Bestimmen einer Spül-Messspannung während des Spülvorgangs;
• Vergleichen des zeitlichen Verhaltens der Spül-Messspannung mit einem vorgegebenen zweiten Membran-Grenzwertbereichs;
• Erzeugen einer vierten Fehlermeldung, welche eine Fehlfunktion der Membran im Gassensor anzeigt, wenn die Messspannung ausserhalb des ersten Membran-Grenzwertbereichs und die Spül-Messspannung ausserhalb des zweiten Membran-Grenzwertbereichs liegen.

7. Verfahren nach einem der Ansprüche 1 bis 6, **dadurch gekennzeichnet, dass** die erste, zweite, dritte und/oder vierte Fehlermeldung in einer Steuereinheit des Gassensors registriert wird.

8. Verfahren nach einem der Ansprüche 1 bis 7, **dadurch gekennzeichnet, dass** die erste, zweite, dritte und/oder vierte Fehlermeldung optisch, akustisch und/oder elektronisch angezeigt wird.

## Claims

1. A method for functionality monitoring of a gas sensor which works based on the principle of thermal conductivity, wherein the gas sensor includes a measuring chamber (6) having a purge gas port (2), a thermal conductivity sensor (7) disposed in the measuring chamber (6) for determining one or more measured values, a temperature sensor (14), and a membrane (8), which separates the measuring chamber (6) from a fluid medium (13) when in operation and is permeable to a target gas; and wherein the method includes the following steps:
a. performing a calibration cycle in a calibration medium, which is a fluid medium having a known target gas concentration, wherein the calibration cycle includes a purging process and a subsequent measuring process, and determining a calibration baseline which includes the calibration voltage as a function of the voltage values determined during the purging process;
b. performing a measuring cycle in a measuring medium, which is a fluid medium having an unknown target gas concentration, wherein the measuring cycle includes a purging process and a subsequent measuring process, and determining a calibration baseline which includes the calibration voltage as a function of the voltage values determined during the purging process;
c. calculating a baseline comparison value by including the calibration baseline or the measuring baseline;
d. comparing the baseline comparison value to a predetermined baseline threshold value, and, if the amount of the baseline comparison value is greater than the baseline threshold value, generating a first error message which indicates a malfunction of the purge gas supply,
wherein a purging process includes that the measuring chamber (6) is purged with a purge gas; wherein a measuring process includes that the membrane (8) of the gas sensor is brought into contact with the fluid medium, that target gases contained in the fluid medium diffuse through the membrane (8) into the measuring chamber (6), and that one or more measured values are determined using a thermal conductivity sensor (7) disposed in the measuring chamber (6).

2. The method according to claim 1, **characterized in that** it further includes the following steps:
• performing a measuring cycle in a measuring medium and determining a measuring voltage during the measuring process using an electronic measuring circuit of the gas sensor;
• comparing the measuring voltage to a predetermined voltage range, and
i. generating another error message if the measuring voltage is outside of the voltage range;
or
ii. determining a measuring current intensity if the measuring voltage is within the voltage range and determining a first control value by comparing the measuring current intensity to a predetermined measuring current intensity;
determining a control current intensity using a control circuit, which is a part of the measuring circuit, and determining a second control value by comparing the measuring current intensity to the control current intensity;
comparing the first control value to a first control threshold value and the second control value to a second control threshold value; and
generating a second error message, which indicates a malfunction of the gas sensor if the first control value is greater than the first control threshold value and/or the second control value is greater than the second control threshold value.

3. The method according to claim 2, **characterized in that** it further includes the following steps:
• comparing the second control value to a third control threshold value if the second control value is greater than the second threshold value; and
• generating a third error message, which indicates a malfunction of sensor failure if the second control value is greater than the third control threshold value.

4. The method according to claim 1, **characterized in that** it further includes the following steps:
• performing a measuring cycle in a measuring medium and determining a measuring voltage during the measuring process using an electronic measuring circuit of the gas sensor;
• determining a measuring current intensity and adjusting an adjustable first resistor or the supply voltage of the electronic measuring circuit while determining a value of a manipulated variable, such that the measuring current intensity matches a predetermined measuring current intensity;
• comparing the value of the manipulated variable to a predetermined first value of a manipulated control variable and generating a second error message, which indicates a malfunction of the gas sensor if the value of the manipulated variable is greater than the value of the first manipulated control variable.

5. The method according to claim 4, **characterized in that** it further includes the following steps:
• comparing the value of the manipulated variable to the value of a second manipulated control variable if the value of the manipulated variable is greater than the value of the first manipulated control variable; and
• generating a third error message, which indicates a failure of the gas sensor if the value of the manipulated variable is greater than the first and greater than the second value of the second manipulated control variable.

6. The method according to any one of claims 1 to 5, **characterized in that** it further includes the following steps:
• performing a measuring cycle and determining the measuring voltage as a function of time during the measuring process;
• comparing the behavior of the measuring voltage over time to a predetermined first membrane threshold range;
• performing a purging process and determining a purging measuring voltage during the purging process;
• comparing the behavior of the purging measuring voltage over time to a predetermined second membrane threshold value range;
• generating a fourth error message, which indicates a malfunction of the membrane in the gas sensor if the measuring voltage is outside the first membrane threshold value range and the purging measuring voltage is outside the second membrane threshold value range.

7. The method according to any one of claims 1 to 6, **characterized in that** the first, second, third, and/or fourth error message(s) is/are registered in a control unit of the gas sensor.

8. The method according to any one of claims 1 to 7, **characterized in that** the first, second, third, and/or fourth error message(s) is/are displayed optically, acoustically and/or electronically.

## Revendications

1. Procédé de contrôle du fonctionnement d'un capteur de gaz, lequel fonctionne selon le principe de la conductivité thermique, dans lequel le capteur de gaz comprend une chambre de mesure (6) avec un raccord de gaz de rinçage (2), une sonde de conductivité thermique (7) agencée dans la chambre de mesure (6) pour la détermination d'une ou plusieurs valeurs de mesure, une sonde de température (14), et une membrane (8), laquelle sépare en fonctionnement la chambre de mesure (6) d'un milieu fluide (13) et est perméable à un gaz cible ; et dans lequel le procédé comprend les étapes suivantes :
a. réalisation d'un cycle de calibrage dans un milieu de calibrage, qui est un milieu fluide avec concentration de gaz cible connue, dans lequel le cycle de calibrage comprend une opération de rinçage et une opération de mesure subséquente, et détermination d'une ligne de base de calibrage, laquelle comprend la tension de calibrage en fonction des valeurs de tension déterminées pendant l'opération de rinçage ;
b. réalisation d'un cycle de mesure dans un milieu de mesure, qui est un milieu fluide avec concentration de gaz cible inconnue, dans lequel le cycle de mesure comprend au moins une opération de rinçage et une opération de mesure subséquente, et détermination d'une ligne de base de mesure, laquelle comprend la tension de mesure en fonction des valeurs de tension déterminées pendant l'opération de rinçage ;
c. calcul d'une valeur de comparaison de ligne de base en tenant compte de la ligne de base de calibrage ou de la ligne de base de mesure ;
d. comparaison de la valeur de comparaison de ligne de base avec une valeur limite de ligne de base prédéfinie, et lorsque la valeur de la valeur de comparaison de ligne de base est supérieure à la valeur limite de ligne de base, génération d'un premier message d'erreur, lequel affiche un dysfonctionnement dans l'amenée de gaz de rinçage,
dans lequel une opération de rinçage comprend le fait que la chambre de mesure (6) est rincée avec un gaz de rinçage ; dans lequel une opération de mesure comprend le fait que la membrane (8) du capteur de gaz est mise en contact avec le milieu fluide, que cela diffuse des gaz cible contenus dans le milieu fluide par la membrane (8) dans la chambre de mesure (6), et qu'une ou plusieurs valeurs de mesure sont déterminées avec une sonde de conductivité thermique (7) agencée dans la chambre de mesure (6).

2. Procédé selon la revendication 1, **caractérisé en ce que** celui-ci comprend en outre les étapes suivantes :
• réalisation d'un cycle de mesure dans un milieu de mesure et pendant l'opération de mesure, détermination d'une tension de mesure avec un circuit de mesure électronique du capteur de gaz ;
• comparaison de la tension de mesure avec une plage de tension prédéfinie et
i. génération d'un autre message d'erreur, lorsque la tension de mesure se situe à l'extérieur de la plage de tension
ou
ii. détermination d'une intensité de mesure, lorsque la tension de mesure se situe à l'intérieur de la plage de tension, et détermination d'une première valeur de contrôle par comparaison de l'intensité de mesure avec une intensité de mesure prédéfinie ;
détermination d'une intensité de contrôle par le biais d'un circuit de contrôle, lequel est une partie du circuit de mesure, et détermination d'une deuxième valeur de contrôle par comparaison de l'intensité de mesure avec l'intensité de contrôle ;
comparaison de la première valeur de contrôle avec une première valeur limite de contrôle et de la deuxième valeur de contrôle avec une deuxième valeur limite de contrôle, et
génération d'un deuxième message d'erreur, lequel affiche un dysfonctionnement du capteur de gaz, lorsque la première valeur de contrôle est supérieure à la première valeur limite de contrôle et/ou la deuxième valeur de contrôle est supérieure à la deuxième valeur limite de contrôle.

3. Procédé selon la revendication 2, **caractérisé en ce que** celui-ci comprend en outre les étapes suivantes :
• comparaison de la deuxième valeur de contrôle avec une troisième valeur limite de contrôle, lorsque la deuxième valeur de contrôle est supérieure à la deuxième valeur limite ; et
• génération d'un troisième message d'erreur, lequel affiche un dysfonctionnement panne du capteur de gaz, lorsque la deuxième valeur de contrôle est supérieure à la troisième valeur limite de contrôle.

4. Procédé selon la revendication 1, **caractérisé en ce que** celui-ci comprend en outre les étapes suivantes :
• réalisation d'un cycle de mesure dans un milieu de mesure et détermination d'une tension de mesure pendant l'opération de mesure avec un circuit de mesure électronique du capteur de gaz ;
• détermination d'une intensité de mesure, et réajustage d'une première résistance réglable ou de la tension d'alimentation du circuit de mesure électronique en déterminant une valeur de réglage, de sorte que l'intensité de mesure correspond à une intensité de mesure prédéfinie ;
• comparaison de la valeur de réglage avec une première valeur de réglage de contrôle prédéfinie et génération d'un deuxième message d'erreur, lequel affiche un dysfonctionnement du capteur de gaz, lorsque la valeur de réglage est supérieure à la première valeur de réglage de contrôle.

5. Procédé selon la revendication 4, **caractérisé en ce que** celui-ci comprend en outre les étapes suivantes :
• comparaison de la valeur de réglage avec une deuxième valeur de réglage de contrôle, lorsque la valeur de réglage est supérieure à la première valeur de réglage de contrôle ; et
• génération d'un troisième message d'erreur, lequel affiche une panne du capteur de gaz, lorsque la valeur de réglage est supérieure à la première valeur de réglage de contrôle et supérieure à la deuxième valeur de réglage de contrôle.

6. Procédé selon l'une quelconque des revendications 1 à 5, **caractérisé en ce que** celui-ci comprend en outre les étapes suivantes :
• réalisation d'un cycle de mesure et détermination de la tension de mesure en fonction du temps pendant l'opération de mesure ;
• comparaison du comportement temporel de la tension de mesure avec une première plage de valeur limite de membrane prédéfinie ;
• réalisation d'une opération de rinçage et détermination d'une tension de mesure de rinçage pendant l'opération de rinçage ;
• comparaison du comportement temporel de la tension de mesure de rinçage avec une deuxième plage de valeur limite de membrane prédéfinie ;
• génération d'un quatrième message d'erreur, lequel affiche un dysfonctionnement de la membrane dans le capteur de gaz, lorsque la tension de mesure se situe à l'extérieur de la première plage de valeur limite de membrane et la tension de mesure de rinçage se situe à l'extérieur de la deuxième plage de valeur limite de membrane.

7. Procédé selon l'une quelconque des revendications 1 à 6, **caractérisé en ce que** le premier, deuxième, troisième et/ou quatrième message d'erreur est enregistré dans une unité de commande du capteur de gaz.

8. Procédé selon l'une quelconque des revendications 1 à 7, **caractérisé en ce que** le premier, deuxième, troisième et/ou quatrième message d'erreur est affiché sous forme optique, acoustique et/ou électronique.
